# EUROPÄISCHE PATENTSCHRIFT

(11) **EP 1 105 057 B1**
(45) Veröffentlichungstag und Bekanntmachung des Hinweises auf die Patenterteilung: **15.05.2002**
(21) Anmeldenummer: 00943907.6
(22) Anmeldetag: 27.06.2000
(51) Int. Cl.: A61B 17/70

(54) **KNOCHENSCHRAUBE**
BONE SCREW
VIS POUR FRACTURE OSSEUSE

(30) Priorität: 02.08.1999 DE 19936286
(43) Veröffentlichungstag der Anmeldung: 13.06.2001
(73) Patentinhaber: Biedermann, Lutz, 78048 Villingen-Schwenningen (DE); Harms, Jürgen, Prof. Dr., 76227 Karlsruhe (DE)
(72) Erfinder: Biedermann, Lutz, 78048 Villingen-Schwenningen (DE); Harms, Jürgen, Prof. Dr., 76227 Karlsruhe (DE)
(74) Vertreter: Prüfer, Lutz H., Dipl.-Phys.
(86) Internationale Anmeldenummer: EP0005966
(87) Internationale Veröffentlichungsnummer: WO0108574

(56) Entgegenhaltungen:
- WO-A-98/32386
- FR-A- 2 697 992
- US-A- 5 443 467
- US-A- 5 624 442

## Beschreibung

Die Erfindung betrifft eine Knochenschraube nach dem Oberbegriff des Patentanspruches 1.

Eine derartige Knochenschraube ist aus der EP 0 614 649 A bekannt. Bei dieser ist zum perfekten Verriegeln der Stab-Knochenschrauben-Verbindung eine in die offene Bohrung einzuschraubende Sicherungsmutter vorgesehen.

Aufgabe der Erfindung ist es, eine Knochenschraube der eingangs beschriebenen Art zu schaffen, die ohne eine solche Innenmutter auskommt.

Diese Aufgabe wird durch die in Patentanspruch 1 gekennzeichnete Knochenschraube gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren.

Von den Figuren zeigen:
- Fig. 1: eine explosionsartige Seitenansicht einer ersten Ausführungsform einer Knochenschraube;
- Fig. 2: eine Schnittdarstellung durch die Knochenschraube;
- Fig. 3: eine explosionsartige Darstellung einer mit der Knochenschraube zu verwendenden Außenmutter in vergrößertem Maßstab, teilweise geschnitten;
- Fig. 4: die Außenmutter in zusammengesetztem Zustand vor der Einwirkung auf einen Stab;
- Fig. 5: die selbe Darstellung nach der Einwirkung auf den Stab;
- Fig. 6: eine der Fig. 1 entsprechende explosionsartige Seitenansicht einer zweiten Ausführungsform der Knochenschraube;
- Fig. 7: eine Schnittdarstellung durch die Knochenschraube der zweiten Ausführungsform;
- Fig. 8: eine der Fig. 1 entsprechende Seitenansicht einer dritten Ausführungsform der Knochenschraube; und
- Fig. 9: eine Schnittdarstellung durch die dritte Ausführungsform.

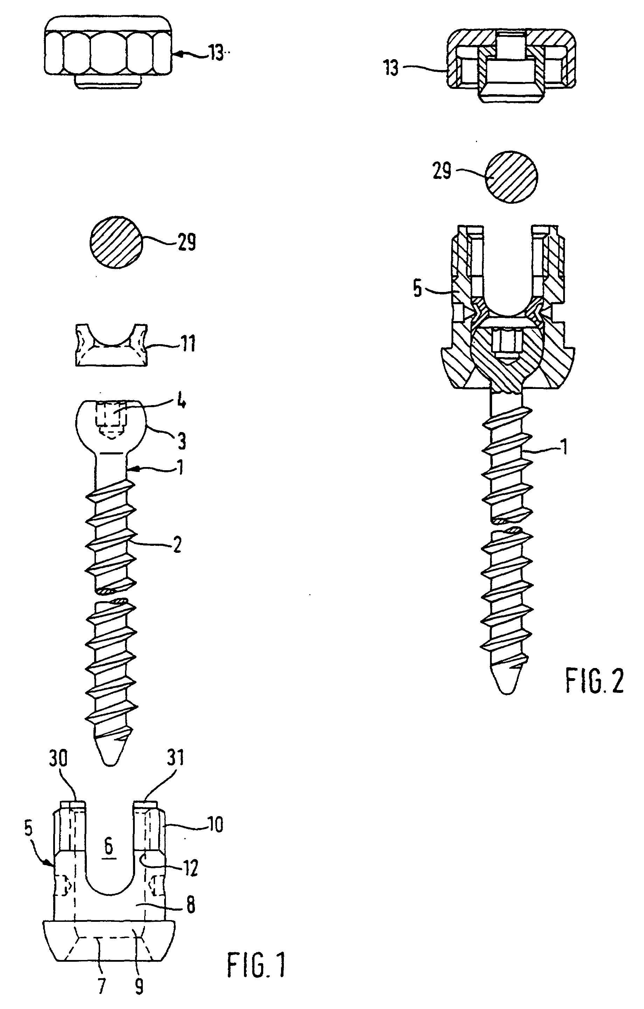

Die Knochenschraube nach der in den Fig. 1 und 2 gezeigten Ausführungsform weist das eigentliche Schraubenelement 1 mit einem Gewindeabschnitt 2 und einem Kopf 3 auf. Der Kopf ist angrenzend an den Gewindeabschnitt kugelsegmentförmig ausgebildet. Koaxial zur Gewindeachse und auf dem dem Gewindeabschnitt 2 gegenüberliegenden Ende weist der Kopf eine Ausnehmung 4 zum Ineingriffbringen mit einem Inbusschlüssel auf.

Die Knochenschraube umfaßt ferner ein zylindrisch ausgebildetes Aufnahmeteil 5. Dieses weist an seinem einen Ende eine axialsymmetrisch ausgerichtete erste Bohrung 7 auf, deren Durchmesser größer als der des Gewindeabschnittes 2 und kleiner als der des Kopfes 3 ist. Das Aufnahmeteil 5 weist ferner eine koaxiale zweite Bohrung 8 auf, die auf dem der ersten Bohrung 7 gegenüberliegenden Ende offen ist und deren Durchmesser so groß ist, daß das Schraubenelement 1 durch das offene Ende mit seinem Gewindeabschnitt 2 durch die erste Bohrung 7 hindurch und mit dem Kopf 3 bis zum Grund der zweiten Bohrung führbar ist. Zwischen der ersten und der zweiten Bohrung ist ein kleiner koaxialer Abschnitt 9 vorgesehen, der unmittelbar an die erste Bohrung angrenzt und zum offenen Bereich hin sphärisch ausgebildet ist, wobei der Radius im wesentlichen gleich dem Radius des kugelsegmentförmigen Abschnittes des Kopfes 3 ist. Ferner weist das Aufnahmeteil 5 eine zur Mitte des Teiles symmetrisch angeordnete U-förmige Ausnehmung 6 auf, deren Grund zu der ersten Bohrung 7 hin gerichtet ist und deren beide Seitenschenkel 30, 31 sich zu dem der ersten Bohrung 7 abgewandten offenen Ende hin erstrecken. Am freien Ende der Schenkel der U-förmigen Ausnehmung ist ein Außengewinde 10 vorgesehen.

Auf der am freien Ende des Kopfes 3 liegenden Seite befindet sich eine Druckscheibe 11, die sich so ausgebildet ist, daß sie auf ihrer dem Kopf 3 zugewandten Seite eine sphärische Absenkung aufweist, deren Radius im wesentlichen gleich dem Radius des kugelsegmentförmigen Abschnittes des Kopfes ist. Der Außendurchmesser der Druckscheibe 11 ist so gewählt, daß diese in den Zylinderabschnitt 12 der zweiten Bohrung 8 eine Gleitbewegung ausführen kann, also in dem Zylinderabschnitt zu dem Kopf hin verschiebbar ist. Die Druckscheibe weist eine koaxiale Bohrung auf, die einen Zugriff zur Ausnehmung 4 ermöglicht.

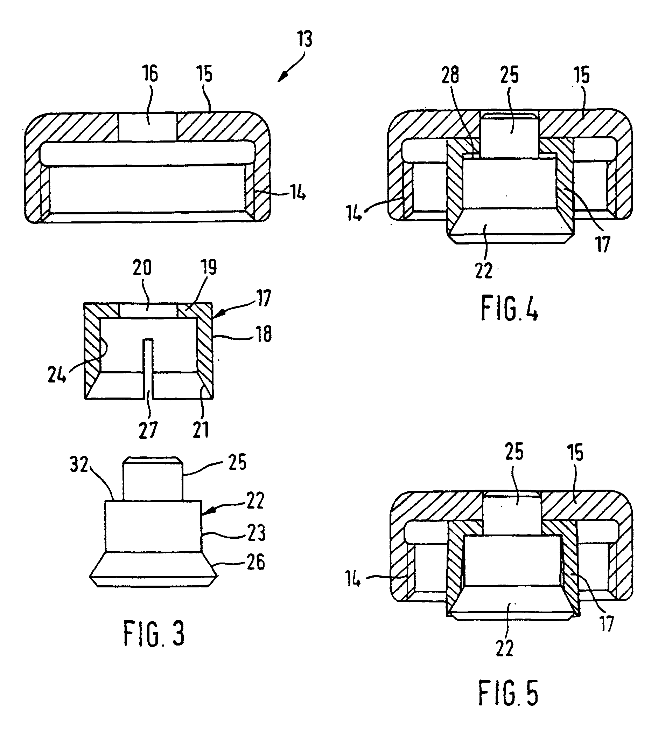

Die Knochenschraube umfaßt ferner eine Außenmutter 13, die im weiteren im Detail insbesondere anhand der Figuren 3 bis 5 erläutert wird. Die Außenmutter ist als eine Hutmutter ausgebildet und weist einen üblichen seitlichen Gewindeabschnitt 14, der die eigentliche Mutter bildet, und einen an der dem Gewindeabschnitt abgewandten Außenseite des Gewindeabschnittes anschließenden Deckelteil 15 auf. Der Deckelteil ist wie bei einer üblichen Hutmutter ausgebildet, weist aber zusätzlich eine konzentrische Bohrung 16 mit einem ersten Durchmesser auf. Es ist ferner eine Hülse 17 vorgesehen, die einen hohlzylinderförmigen Mantel 18 und auf ihrer dem Deckelteil 15 zugewandten Seite einen Boden 19 aufweist. In dem Boden 19 ist eine konzentrische Bohrung 20 mit einem zweiten Durchmesser vorgesehen. Der zweite Durchmesser ist gleich dem ersten Durchmesser der Bohrung 16 oder ein wenig größer. Wie am besten aus Fig. 3 ersichtlich ist, weist der hohlzylindrische Mantel 18 an seinem dem Boden 19 abgewandten freien Ende auf seiner Innenseite einen Rand 21 auf, der nach außen hin so abgeschrägt ist, daß er die Form eines Kegelstumpfes aufweist, wobei der Neigungswinkel der Schräge gegenüber der Zylinderinnenwand etwa 30 bis 60° und vorzugsweise etwa 40° beträgt. Der Außendurchmesser der zylindrischen Hülse 17 ist nahezu gleich dem Durchmesser der zweiten Bohrung 8 und um soviel kleiner als letztere, daß die Hülse gerade ohne Reibung in die zweite Bohrung 8 einführbar ist.

Zusätzlich ist ein Druckelement 22 vorgesehen. Dieses weist einen zweiten Abschnitt 23 auf, der zylindrisch ausgebildet ist. Der Außendurchmesser des Zylinders ist im wesentlichen gleich dem Innendurchmesser des Zylindermantels 18. Er ist so bemessen, daß das Druckelement durch Einführen des zweiten Abschnittes in das Innere des Mantels 18 durch Reibungskraft in diesem gehalten wird. Die Höhe des zweiten Abschnittes in Axialrichtung gesehen ist, wie am besten aus Fig. 4 ersichtlich ist, ein wenig kleiner als die entsprechende Länge des zylindrischen Abschnittes 24 des Mantels 18. Die Differenz liegt vorzugsweise im Bereich von 0,25 bis 0,5 Millimeter. Auf der dem Deckelteil zugewandten Seite ist der zweite Abschnitt plan ausgebildet und weist einen konzentrisch angeordneten stiftförmigen Ansatz 25 auf. Der Durchmesser dieses Ansatzes entspricht im wesentlichen dem Durchmesser der Bohrung 16 und ist so bemessen, daß der Stift in der Bohrung 16 aufgrund der Reibungskraft in dieser gehalten wird. Auf der dem Deckelteil 15 abgewandten Seite weist der zweite Abschnitt 23 einen daran anschließenden ersten Abschnitt 26 auf. Dieser ist kegelstumpfförmig ausgebildet derart, daß der kleine Innendurchmesser dem Durchmesser des zweiten Abschnittes 23 entspricht und daß der Durchmesser der dem zweiten Abschnitt abgewandten Oberfläche gleich dem Durchmesser des daran anliegenden Teiles des abgeschrägten Randes 21 ist. Der Winkel des Kegelmantels ist vorzugsweise gleich dem Winkel des inneren Randes 21.

In dem in Fig. 4 gezeigten vormontierten Zustand entsteht so zwischen dem Boden 19 der Hülse 17 und der diesem zugewandten Bodenfläche 32 ein spaltförmiger Abstand 28.

Wie am besten aus Fig. 3 ersichtlich ist, weist die Hülse 17 an ihrem dem Deckelteil abgewandten Rand einen sich parallel zur Symmetrieachse der Hülse erstreckenden Schlitz 27 auf, der eine Ausdehnung des freien Randes der Hülse ermöglicht.

Im Betrieb sind zunächst das Schraubenelement 1, das Aufnahmeteil 5 und die Druckscheibe in der ansich bekannten Weise zusammengesetzt, wie dies am besten aus Fig. 2 ersichtlich ist. Dann wird der mit der Knochenschraube zu verbindende Stab 29 eingesetzt. Die Außenmutter ist in dem in Fig. 4 ersichtlichen Zustand vormontiert, das heißt, das Druckelement 22 ist in die Hülse 17 eingesetzt und zusammen mit dieser dadurch mit dem Deckelteil 15 verbunden, daß der Ansatz 25 in die Bohrung 16 eingedrückt wird. Die so vormontierte Mutter wird nun auf das Außengewinde des Aufnahmeteils 5 aufgeschraubt. Dabei liegt der Stab 29 einerseits auf der Druckscheibe 11 auf. Andererseits wird er durch das beim Aufschrauben mit dem Stab in Kontakt gelangende Druckelement 22 mit Druck beaufschlagt, sobald die Außenmutter 13 die Endposition nahezu erreicht hat. Beim weiteren Aufschrauben der Außenmutter in die gewünschte Endposition wird das Druckelement 22 in der aus Fig. 5 ersichtlichen Weise bis zum Grund der Hülse 17 geschoben, was gleichzeitig zur Folge hat, daß der erste Abschnitt 26 so auf das freie Ende der Hülse 17 einwirkt, die Hülse bzw. deren freies Ende in der in Fig. 5 gezeigten Weise ein wenig nach außen gedrückt wird. Dadurch wird erreicht, daß der Mantel 18 mit seiner Außenfläche wiederum eine Kraft auf die freien Schenkel 30, 31 in dem Bereich des Außengewindes 10 so ausüben, daß diese freien Schenkel 30, 31 wiederum den Gewindeabschnitt 10 in den das Innengewinde aufweisenden Abschnitt 14 hineindrücken. Dadurch wird eine Verriegelung bewirkt, die ein unbeabsichtigtes Lösen der Außenmutter 13 verhindert.

In dem oben beschriebenen Ausführungsbeispiel handelt es sich um eine sogenannte Polyaxialschraube, bei der das Schraubenelement 1 und das Aufnahmeteil 5 winkelmäßig relativ zueinander bewegbar sind. In einer abgewandelten Ausführungsform sind das Schraubenelement 1 und ein den Stab 29 aufnehmendes Aufnahmeteil einstückig miteinander ausgebildet, etwa derart, daß in der in Fig. 2 gezeigte Weise das Aufnahmeteil 5, der Kopf 3 und die Druckscheibe 11 einstückig ausgebildet sind. Die Außenmutter 13 weist in diesem Fall die identische oben beschriebene Form auf. In der Betriebsweise erfolgt die Arretierung der Außenmutter durch Einwirken der Kraft vom Stab 29 auf das Druckelement 22 in der oben beschriebenen Weise, so daß die gleiche Verriegelung erzielt wird.

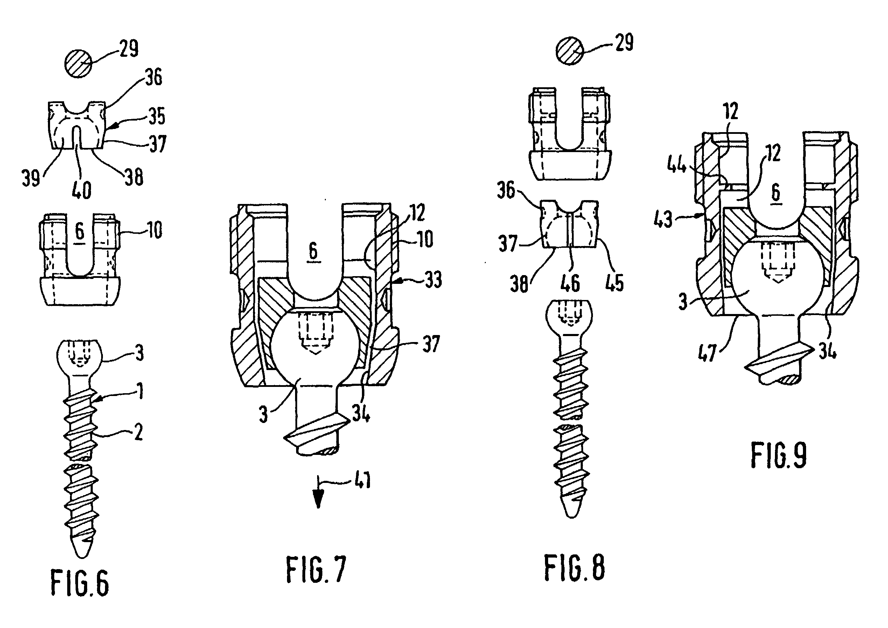

Die in den Fig. 6 und 7 gezeigte zweite Ausführungsform unterscheidet sich von der ersten Ausführungsform in der Ausbildung des Aufnahmeteiles. Wie bei der ersten Ausführungsform weist die zweite Bohrung einen Zylinderabschnitt 12 auf. Anstelle der ersten Bohrung 7 und des sphärischen Abschnittes 9 ist ein Abschnitt mit einer sich vom zweiten Abschnitt weg kegelförmig verjüngender Abschnitt 34 vorgesehen. Anstelle des Druckelements 11 ist ein Druckelement 35 vorgesehen, welches einen zylindrischen Abschnitt 36 und einen daran anschließenden kegelsegmentförmigen Abschnitt 37 aufweist. Auf der von dem kegelsegmentförmigen Abschnitt 37 umgebenen Endfläche 38 weist das Element eine kugelsegmentförmige Ausnehmung 39 auf, deren Durchmesser annähernd gleich bzw. gleich dem Außendurchmesser des Kopfes 3 ist. Die Wandung des Abschnittes 37 weist, wie in Fig. 6 gezeigt ist, einen von der Endfläche 38 sich in axialer Richtung erstreckenden Schlitz 40 auf. Die Außenabmessung des kegelsegmentförmigen Abschnittes 37 ist so gewählt, daß der Durchmesser an dem am zylindrischen Abschnitt 36 angrenzenden Teil größer ist als der Durchmesser des Abschnittes 34 an seinem freien offenen Ende. Gleichzeitig ist die Neigung des kegelsegmentförmigen Abschnittes 37 im wesentlichen gleich der Neigung des Abschnittes 34 gewählt. Ferner ist der Durchmesser des kegelsegmentförmigen Abschnittes 37 an seiner an dem zylindrischen Abschnitt 36 angrenzenden Seite gleich dem Durchmesser des zylindrischen Abschnittes 36 und kleiner oder nahezu gleich dem Innendurchmesser des Zylinderabschnittes 12.

Im Betrieb wird das Schraubenelement 1 von der vom freien Ende des Abschnittes 34 gebildeten Öffnung durch die Öffnung 34 und den Zylinderabschnitt 12 hindurchgeschoben und in das Druckelement 35 von der Endfläche 38 her in die Ausnehmung 39 eingedrückt. Anschließend wird das Schraubenelement mit dem so aufgebrachten Druckelement in die in Fig. 7 gezeigte Stellung und dann in Richtung des Pfeiles 41 soweit bewegt, bis der kegelsegementförmige Abschnitt 37 leicht an dem Abschnitt 34 anliegt. Die äußere Form des Aufnahmeteiles 33 stimmt in allen Merkmalen und insbesondere in dem Außengewinde 10 mit der ersten Ausführungsform überein. Die mit dieser Ausführungsform verwendbare Außenmutter ist mit der in den Fig. 3 und 4 gezeigten Ausführungsform identisch.

Im Betrieb wird der Stab 29 wie bei der ersten Ausführungsform in die U-förmige Ausnehmung 6 eingelegt und mittels der Außenmutter 13 fixiert. Die Abmessungen der Außenmutter relativ zum Durchmesser des Zylinderabschnittes 12 entsprechen der ersten Ausführungsform.

Bei der in den Fig. 8 und 9 gezeigte dritten Ausführungsform stimmt das Aufnahmeteil 43 mit einer Ausnahme mit dem Aufnahmeteil 33 überein. Diese Ausnahme besteht darin, daß in dem Zylinderabschnitt 12 in einem vorgegebenen Abstand vom freien Ende des Zylinderabschnittes ein ringsegmentförmiger Anschlag 44 vorgesehen ist.

Ein Druckelement 45 stimmt in allen Merkmalen mit dem Druckelement 35 überein mit der Ausnahme, daß anstelle des nicht durchgehenden Schlitzes 40 hier ein Schlitz 46 vorgesehen ist, der sich von dem an die Endfläche 38 angrenzenden Ende bis zum gegenüberliegenden Ende erstreckt. Ansonsten stimmen auch die Relativabmessungen des zylinderförmigen Abschnittes und des kegelsegmentförmigen Abschnittes 37 mit den Innenabmessungen vom Zylinderabschnitt 12 und dem Abschnitt 34 überein.

Der Schlitz 46 ist so breit gewählt, daß das Druckelement 45 in Umfangsrichtung so weit zusammendrückbar ist, daß es von der Unterseite 47 des Abschnittes 34 her in den Zylinderabschnitt 12 einschiebbar und bis zum Anschlag 44 bewegbar ist. Der Außendurchmesser des kegelsegmentförmigen Abschnittes 37 ist an seinem freien Ende so gewählt, daß er in der Anschlagposition um so viel kleiner als der Innendurchmesser der angrenzenden Wandung ist, daß ein Aufweiten des kegelsegmentförmigen Abschnittes 37 derart möglich ist, daß der Kopf 3 in die Unterseite 47 eingeführt und in das Druckelement 45 eindrückbar ist und von diesem in gleicher Weise wie bei dem zweiten Ausführungsbeispiel gehalten wird. Nach dem Hineindrücken des Kopfes 3 hat das Druckelement 45 relativ zu den Innenabmessungen des Aufnahmeteiles 43 die gleichen Abmessungen wie bei der zweiten Ausführungsform.

Im Betrieb wird der Stab 29 wie bei den vorherigen Ausführungsbeispielen in die U-förmige Ausnehmung 6 eingelegt. Anschließend wird die Außenmutter wie bei den vorherigen Ausführungsbeispielen aufgeschraubt und dadurch eine Arretierung zwischen Aufnahmeteil 43 und Schraubenelement 1 erreicht.

## Patentansprüche

1. Knochenschraube mit einem Gewindeabschnitt (2) und einem kopfseitigen zylindrischen Aufnahmeteil (5) für die Aufnahme eines mit der Knochenschraube zu verbindenden Stabes (29), wobei das Aufnahmeteil eine offene Bohrung (8) und einen im wesentlichen U-förmigen Querschnitt mit zwei freien, ein Außengewinde (10) besitzenden Schenkeln (30, 31) aufweist, und mit einer auf das Außengewinde (10) aufschraubbaren Außenmutter (13),
**dadurch gekennzeichnet, daß** die Außenmutter (13) im Inneren ein ein vorgegebenes Innenmaß aufweisendes hülsenförmiges Element (17), dessen Außendurchmesser nahezu gleich oder wenig kleiner als der Durchmesser der Bohrung (8) ist, und ein darin angeordnetes Druckelement (22) aufweist, wobei das Druckelement (22) an seinem dem Grund der Bohrung (8) zugewandten Ende einen ersten Abschnitt (26) aufweist, dessen Außenmaß größer als das vorgegebene Innenmaß ist und der beim Ausüben von Druck auf den aufnehmenden Stab (29) eine Aufweitung des hülsenförmigen Elementes (17) bewirkt.

2. Knochenschraube nach Anspruch 1, **dadurch gekennzeichnet, daß** die Außenmutter (13) als Hutmutter mit einem seitlichen Gewindeabschnitt (14) und einem am äußeren Rand desselben vorgesehenen Deckelteil (15) ausgebildet ist, das hülsenförmige Element (17) einen sich in axialer Richtung erstreckenden zylindrischen Abschnitt vorgegebener Länge und das Druckelement (22) einen zweiten Abschnitt (23), dessen axiale Länge kleiner als die Länge des zylindrischen Abschnittes ist, aufweisen.

3. Knochenschraube nach Anspruch 2, **dadurch gekennzeichnet, daß** das hülsenförmige Element (17) an seinem dem Deckelteil abgewandten Ende einen nach außen konvexen Wandabschnitt (21) aufweist und der erste Abschnitt (26) des Druckelement (22) an diesem anliegt.

4. Knochenschraube nach einem der Ansprüche 1 bis 3, **dadurch gekennzeichnet, daß** der erste Abschnitt (26) kegelstumpfförmig ausgebildet ist, wobei die Basis auf der dem Grund der Bohrung (8) zugewandten Seite liegt.

5. Knochenschraube nach einem der Ansprüche 2 bis 4, **dadurch gekennzeichnet, daß** das hülseförmige Element (17) und das Druckelement (22) einerseits und das Deckelteil (15) andererseits miteinander verbunden sind.

6. Knochenschraube nach Anspruch 5, **dadurch gekennzeichnet, daß** das Deckelteil (15) eine konzentrische Bohrung (16) und hülseformige Element (17) und Druckelement (22) einen auf der Deckelseite hervorstehenden konzentrischen Ansatz (25), der in der Bohrung (16) gehalten wird, aufweisen.

7. Knochenschraube nach Anspruch 6, **dadurch gekennzeichnet, daß** das hülseförmige Element (17) auf seiner dem Deckelteil (15) zugewandten Seite einen Boden mit einer konzentrischen Bohrung (20) aufweist und der Ansatz (25) an dem deckelseitigen Ende des Druckelements (22) vorgesehen ist.

8. Knochenschraube nach einem der Ansprüche 4 bis 7, **dadurch gekennzeichnet, daß** das freie Ende des hülseförmigen Elementes (17) einen inneren kegelstumpfförmigen Abschnitt (21) aufweist, dessen Neigung im wesentlichen gleich der Neigung des ersten Abschnittes (26) ist.

9. Knochenschraube nach einem der Ansprüche 1 bis 8, **dadurch gekennzeichnet, daß** das hülseförmige Element (17) auf seiner dem freien Ende zugewandten Seite wenigstens einen von dem Ende ausgehenden Schlitz (27) aufweist.

10. Knochenschraube nach einem der Ansprüche 1 bis 9, **dadurch gekennzeichnet, daß** das Druckelement (22) auf seiner dem Grund der Bohrung (8) zugewandten Seiten in axialer Richtung über den seitlichen Gewindeabschnitt hervorsteht.

## Claims

1. A bone screw having a threaded portion (2) and a head-side cylindrical reception part (5) for receiving a rod (29) to be connected with the bone screw, said reception part comprising an open bore hole and an essentially U-shaped cross section having two free legs (30, 31) provided with an exterior thread (10), and an exterior nut (13) which can be screwed onto the exterior thread (10),
**characterized in that** the exterior nut (13) comprises an inside sleeve-shaped element (17) having a predetermined inner dimension and an outer diameter of which being almost equal to or slightly less than the diameter of the bore (8), and a pressure element (22) arranged therein, said pressure element (22) comprising a first section (26) at its end facing the bottom of said bore (8), the outer dimension of said first section being greater than said predetermined inner dimension and said first section causing said element (17) to be expanded when pressure is exerted on said rod (29) to be received.

2. The bone screw as claimed in claim 1, **characterized in that** said exterior nut (13) is formed as a cap nut having a lateral threaded portion (14) and a cover portion (15) provided at the outer edge thereof, said sleeve-shaped element (17) comprising a cylindrical portion of predetermined length extending im an axial direction, and said element (22) comprising a second section (23) having an axial length which is less than the length of said cylindrical portion.

3. The bone screw as claimed in claim 2, **characterized in that** at its end facing away from the cover portion, said sleeve-shaped element (17) comprises a wall portion (21) being convex outwardly, and which is abutted by the first section (26) of said pressure part (22).

4. The bone screw as claimed in any one of claims 1 to 3,
**characterized in that** said first section (26) is formed in a truncated cone shape with the base being located on the side facing the bottom of the bore.

5. The bone screw as claimed in any one of claims 2 to 4,
**characterized in that** said element (17) and said pressure element (22) on the one hand and said cover portion (15) on the other hand are connected with each other.

6. The bone screw as claimed in claim 5, **characterized in that** said cover portion (15) comprises a concentric bore (16) and said element (17) and pressure element (22) comprise a protruding concentric projection (25) at the cover portion side and which is held in the bore (16).

7. The bone screw as claimed in claim 6, **characterized in that** on its side facing the cover portion (15), said element (17) comprises a bottom having a concentric bore (20), and said projection (25) is provided at the cover end of said pressure element (22).

8. The bone screw as claimed in any one of claims 4 to 7,
**characterized in that** the free end of said element (17) comprises an inner truncated cone shaped section (21) having an inclination which is substantially equal to the inclination of said first section (26).

9. The bone screw as claimed in any one of claims 1 to 8,
**characterized in that** on its side facing the free end, said element (17) comprises at least one slit (27) starting from the end.

10. The bone screw as claimed in any one of claims 1 to 9,
**characterized in that** on its side facing the bottom of said bore hole, said pressure element (22) protrudes from the lateral threaded portion in an axial direction.

## Revendications

1. Vis à os possédant une partie filetée (2) et une partie réceptrice cylindrique (5) située du côté de la tête pour recevoir une broche (29) destinée à être reliée à la vis à os, dans laquelle la partie réceptrice présente un trou ouvert (8) et une section sensiblement en forme de U avec deux bras libres (30, 31) possédant une filetage extérieur (10) et avec un écrou extérieur (13) pouvant être vissé sur le filetage extérieur (10), **caractérisée en ce que** l'écrou extérieur (13) présente à l'intérieur un élément en forme de douille (17) présentant une dimension intérieure prédéterminée, dont le diamètre extérieur est presque égal ou légèrement inférieur au diamètre du trou (8) et un élément de compression (22) disposé dans celui-ci, l'élément de compression (22) présentant à son extrémité orientée vers le fond du trou (8) une première partie (26) dont la dimension extérieure est plus grande que la dimension intérieure prédéterminée et qui réalise une expansion de l'élément en forme de douille (17) lors de l'application de compression sur la broche (29) qui le reçoit.

2. Vis à os selon la revendication 1, **caractérisée en ce que** l'écrou extérieur (13) est conçu comme un écrou borgne avec une partie filetée (14) latérale et une partie formant couvercle (15) prévue sur le bord extérieur de celle-ci, et l'élément en forme de douille (17) présente une partie cylindrique s'étendant dans le sens axial et l'élément de compression (22) une deuxième partie (23) dont la longueur axiale est plus petite que la longueur de la partie cylindrique.

3. Vis à os selon la revendication 2, **caractérisée en ce que** l'élément en forme de douille (17) présente à son extrémité opposée à la partie formant couvercle une partie de paroi (21) convexe vers l'extérieur et la première partie (26) de l'élément de compression (22) repose sur celle-ci.

4. Vis à os selon l'une ou l'ensemble des revendications 1 à 3, **caractérisée en ce que** la première partie (26) est tronconique et sa base repose sur le côté orienté vers le fond du trou (8).

5. Vis à os selon l'une ou l'ensemble des revendications 2 à 4, **caractérisée en ce que** l'élément en forme de douille (17) et l'élément de compression (22), d'une part, et la partie formant couvercle (15) d'autre part sont reliés entre eux.

6. Vis à os selon la revendication 5, **caractérisée en ce que** la partie formant couvercle (15) présente un trou concentrique (16) et l'élément en forme de douille (17) et l'élément de compression (22) présentent une saillie concentrique (25) dépassant du côté du couvercle et retenue dans le trou (16).

7. Vis à os selon la revendication 6, **caractérisée en ce que** l'élément en forme de douille (17) présente sur son côté orienté vers la partie formant couvercle (15) un fond avec un trou concentrique (20) et la saillie (25) est prévue à l'extrémité de l'élément de compression (22) orientée vers le couvercle.

8. Vis à os selon l'une ou l'ensemble des revendications 4 à 7, **caractérisée en ce que** l'extrémité libre de l'élément en forme de douille (17) présente une partie intérieure tronconique (21) dont l'inclinaison est sensiblement égale à l'inclinaison de la première partie (26).

9. Vis à os selon l'une ou l'ensemble des revendications 1 à 8, **caractérisée en ce que** l'élément en forme de douille (17) présente du côté orienté vers l'extrémité libre au moins une fente (27) partant de l'extrémité.

10. Vis à os selon l'une ou l'ensemble des revendications 1 à 9, **caractérisée en ce que** l'élément de compression (22) dépasse dans le sens axial au-delà de la partie filetée latérale du côté orienté vers le fond du trou (8).
